# EUROPÄISCHE PATENTSCHRIFT

(11) **EP 2 393 435 B1**
(45) Veröffentlichungstag und Bekanntmachung des Hinweises auf die Patenterteilung: **05.12.2012**
(21) Anmeldenummer: 10803050.3
(22) Anmeldetag: 16.12.2010
(51) Int. Cl.: A61B 17/16, A61C 1/14

(54) **CHIRURGISCHES INSTRUMENT ZUR LÖSBAREN VERBINDUNG EINES HANDSTÜCKES MIT EINEM CHIRURGISCHEN WERKZEUG**
SURGICAL INSTRUMENT FOR DETACHABLY CONNECTING A HANDPIECE TO A SURGICAL TOOL
INSTRUMENT CHIRURGICAL POUR LE RACCORDEMENT AMOVIBLE D'UNE PIÈCE À MAIN À UN OUTIL CHIRURGICAL

(30) Priorität: 23.12.2009 DE 202009017470 U
(43) Veröffentlichungstag der Anmeldung: 14.12.2011
(73) Patentinhaber: Joimax GmbH, 76227 Karlsruhe (DE)
(72) Erfinder: RIES, Wolfgang, 76351 Linkenheim-Hochstetten (DE)
(74) Vertreter: Lempert, Jost
(86) Internationale Anmeldenummer: PCT/EP2010/007715
(87) Internationale Veröffentlichungsnummer: WO 2011/076364

(56) Entgegenhaltungen:
- EP-A1- 1 880 683
- EP-A1- 1 938 757
- WO-A1-2007/002230
- US-A- 5 741 263
- US-A- 5 871 493

## Beschreibung

Die Erfindung betrifft ein chirurgisches Instrument mit einem einen Antrieb aufweisenden Handstück und mit einem chirurgischen Werkzeug nach dem Oberbegriff des Anspruchs 1.

Chirurgische Werkzeuge, welche in ein Handstück eingesetzt werden, sind in unterschiedlichen Ausgestaltungsformen bekannt. Die Handstücke sind üblicherweise mit einem Antrieb verbunden, so dass die Werkzeuge oder Teilbereiche der chirurgischen Werkzeuge antreibbar, z.B. in Drehung versetzbar sind. Derartige chirurgische Werkzeuge können beispielsweise als Werkzeuge für die minimal-invasive Endoskopie, insbesondere im Bereich der Wirbelsäule, ausgebildet sein.

Aus dem Stand der Technik wie z.B. der DE 10 2006 062 421 A1, ist bekannt, dass ein derartiges Handstück mit einer zentrischen Bohrung oder Ausnehmung versehen ist, in welcher ein Kupplungsbereich oder Ansatzbereich des chirurgischen Werkzeuges eingesteckt ist. Dabei gelangen entsprechende Antriebskupplungen des Handstücks in Eingriff mit Kupplungsteilen des chirurgischen Werkzeuges, sodass ein Drehantrieb realisiert wird. Das Einstecken und Verrasten des chirurgischen Werkzeugs in einem Handstück erfolgt dadurch, dass das Handstück mit einer Werkzeugaufnahme versehen ist, mit welcher ein Rastelement des chirurgischen Werkzeugs lösbar in Eingriff bringbar ist. Ein solches Rastelement kann beispielsweise in Form einer Ringnut ausgebildet sein. Das Gegenstück des Rastelements zur Ringnut innerhalb der Werkzeugaufnahme kann beispielsweise in Form eines Hakens, Kugeln oder ähnlichem ausgebildet sein. Beim Einstecken des chirurgischen Werkzeuges in das Handstück kann somit eine Verrastung oder Kupplung erfolgen, die ein unbeabsichtigtes Ablösen des chirurgischen Werkzeugs vom Handstück während des Gebrauchs verhindert. Um das chirurgische Werkzeug aus dem Handstück zu entnehmen, ist beispielsweise eine Betätigungseinrichtung, wie z.B. ein Knopf oder ein Schieber, zu bedienen.

Um eine optimale Kopplung zwischen chirurgischen Werkzeug und dem Antrieb zu gewährleisten sind am Ende des chirurgischen Werkzeuges Kupplungsteile in Form von Nuten oder Nocken ausgebildet, welche in ein Gegenstück des Antriebes eingreifen. Eine solche Kupplung ist aus der DE 69 622 563 T2 bekannt, wobei ein chirurgisches Instrument eine Schnellkupplung aufweist, die eine Steckverbindung in der Art eines Bajonettverschlusses und einen Koppelzapfen proximal des einzusetzenden Werkzeuges umfasst.

Die aus dem Stand der Technik bekannten Vorrichtungen sind jedoch vielfach kompliziert aufgebaut, entsprechend kostenintensiv, aufwendig in der Herstellung und unter operativen Einsatzbedingungen vielfach schlecht betätigbar.

So zeigt die US 5 741 263 ein chirurgisches Instrument mit einem Handstück und einem Werkzeug mit einem Doppelkupplungsmechanismus, bei dem allerdings zum Einführen des Werkzeugs gleichzeitig ein Schieber verschoben werden muss, um das Einführen des Werkzeugs überhaupt zu ermöglichen. Eine solche doppelte Verbindungsbewegung ist in dem entsprechenden Operationssystem nachteilig und unerwünscht.

Die ebenfalls gattungsgemäße US 5 871 493 zeigt ebenfalls ein chirurgisches Element, bei dem allerdings der Kupplungs-Lösemechanismus am Werkzeug in Form eines Betätigungselements ausgebildet ist. Dies macht das Werkzeug zum einen kompliziert und teuer und damit nicht als Einmalwerkzeug ausbildbar, zum anderen ergibt sich hierdurch gerade bei einem mehrfach verwendbaren Werkzeug eine schwierige Reinigungs- und Desinfektionsmöglichkeit, was mit Gefahren für den Patienten verbunden ist.

Schließlich zeigt die EP 1 880 683 ein nichtgattungsgemäßes chirurgisches Element, bei dem an einem Zwischenteil einerseits durch einen quer einschiebbaren Querstift (der verloren gehen kann) ein Werkzeug verbunden werden kann, andererseits am entgegengesetzten Ende ein Antriebselement bestenfalls reibschlüssig, aber nicht formschlüssig einschiebbar ist und damit diese Teile sämtlich als Drehteile über die gesamte Länge hin offen liegen, so dass ein solches Element zwar in einem offenen Hohlraum, wie als Dentalelement im Mundraum eingesetzt werden kann, mit Sicherheit aber nicht als chirurgisches Element durch Körperöffnungen, insbesondere bei minimalinvasiven Eingriffen.

Die WO 2007/002230 A1 wird als Stand der Technik angesehen und betrifft ein chirurgisches Handstück 20 mit einer drehbaren Welle 42 zur Aufnahme eines Kopfes 54 eines chirurgischen Zusatzteils 320 oder Schneidzubehörs 48. Ein Getriebe 36 hat eine Vielzahl von Antriebsköpfen 86, 92 die gleichzeitig bei verschiedenen Geschwindigkeiten rotieren. Eine Kupplungseinheit 44 hat Stifte 184, die sich entlang der Welle bewegen. Die Kupplungseinheit setzt die Position der Stifte, so dass sie an einem der Antriebsköpfe angreifen. Die Welle rotiert derart mit dem Antriebskopf, an dem die Kupplungsstifte eingreifen. Das Werkzeug der D4 ist insbesondere in den Figuren 27 und 27A dargestellt. Es weist einen proximal angeordneten Kupplungskopf 54, einen distalen Arbeitskopf 50 und einen beide verbindenden Schaft 52 auf. Alle, wie auch die Einzelteile des Kupplungskopfes 54 sind dreh- und axialfest miteinander verbunden. Letztere sind, wie sich im Zusammenhang mit Fig. 27A ergibt (von links nach rechts) eine Schulter 305, ein Kopfkörper 304, eine Nut 306, flache Flächen 310 mit gekanteten Flächen 312 und dicht zwischen diesen angeordneten Schlitzen 308 (Fig. 27) sowie ein proximaler axialer mehrkantiger Ansatz 302.

Der Erfindung liegt die Aufgabe zugrunde, ein chirurgisches Instrument zur Kupplung von chirurgischem Werkzeug der eingangs genannten Art zu schaffen, welche bei einfachem Aufbau und kostengünstiger Herstellung sicher und einfach bedienbar ist.

Zur Lösung der genannten Aufgabe sieht die Erfindung ein chirurgisches Instrument mit den kennzeichnenden Merkmalen des Anspruchs 1 vor.

In besonders bevorzugter Ausgestaltung ist hierbei vorgesehen, dass das chirurgische Werkzeug ein hohles zylindrisches Rohr und ein darin befindliches Arbeitsteil mit einem Schaft und einem Werkzeugkopf aufweist, wobei das Arbeitsteil drehbar gelagert ist und zwar im Schaft, so dass beide verbunden sind und zwar drehbar insbesondere überlagern. Weiterhin sieht die Erfindung vor, dass das erste Kupplungsteil proximal am Rohr des Werkzeuges angeordnet ist und, dass das zweite Kupplungsteil drehbar innerhalb des Rohres des Werkzeugs gelagert ist. Somit wird bei einer Benutzung des chirurgischen Instrumentes nicht das komplette Werkzeug gedreht, sondern nur das Arbeitsteil mittels des zweiten Kupplungsteils, das mit der Antriebswelle in Eingriff ist. Die Antriebswelle des Antriebs versetzt hierbei das Arbeitsteil des chirurgischen Werkzeuges mittels des zweiten Kupplungsteils in Bewegung, wie z.B. in Drehung. Die vom Arbeitsteil getrennte lösbare Verbindung eines Rohres mit einem Handstück, welcher sich demnach nicht dreht, dient als Schutz für das Arbeitsteil bzw. der äußeren Umgebung des Arbeitsbereiches, wie z.B. empfindliches Nervengewebe.

In einer besonders bevorzugten Ausgestaltung ist vorgesehen, dass das Handstück im Wesentlichen zylindrisch ausgebildet ist, wobei das Handstück einen Griff mit einer Aufnahme für das Werkzeug und eine Klemmbefestigung innerhalb der Werkzeugaufnahme aufweist und, dass im Verschlussring des Handstücks eine Ringdichtung zur Abdichtung der Aufnahme des Werkzeugs vorgesehen ist. Weiterhin ist in einer bevorzugten Ausgestaltung vorgesehen, dass am Handstück ein Betätigungsschieber angeordnet ist, der mittels einer ringförmigen Anpressplatte mit wenigstens einer Feder vorzugsweise drei oder vier Federn beaufschlagt ist, wobei der Betätigungsschieber in eine Verriegelungsposition und eine Freigabeposition axial verschiebbar ist.

Eine zuverlässige und schnelle Verrastung des chirurgischen Werkzeuges wird dadurch erreicht, dass der Durchmesser der Werkzeugaufnahme dem der auswechselbaren chirurgischen Werkzeuge angepasst ist, welche ein rundes Rohr mit einem ersten Kupplungsteil aufweisen, welches das Einführen mit geringem Spiel in die Werkzeugaufnahme gestattet. Hierzu ist in einer besonders bevorzugten Ausgestaltung vorgesehen, dass das erste Kupplungsteil proximal am Werkzeug eine Ringnut zur Befestigung und Verriegelung aufweist. Für eine Verrastung ist erfindungsgemäß eine Klemmbefestigung vorgesehen, wobei mindestens ein Klemmstifte, in länglichen radial schräg in die Innenwand der Aufnahme eingefrästen Nuten liegend, kreissehnenförmig in das Innere der Aufnahme ragen. Die Klemmstifte sind hierbei so bemessen, dass sie in einer Verriegelungsposition in die Ringnut des ersten Kupplungsteils formschlüssig eingreifen. Zur Verbindung des chirurgischen Werkzeuges innerhalb des Handstückes mit der Antriebswelle des Antriebs, vorzugsweise eines Elektromotors, ist vorgesehen, dass das zweite Kupplungsteil im Wesentlichen zylindrisch mit einer axialen Bohrung ausgebildet ist und, dass das zweite Kupplungsteil radial eine durchgängige Bohrung hat, die senkrecht zur axialen Bohrung angeordnet ist und diese abschließt. Der Schaft des Werkzeugteils ist in die axiale Bohrung kraft- und/oder stoffschlüssig einsteckbar.

In einer besonders bevorzugten Ausgestaltung ist vorgesehen, dass im proximalen Endbereich des zweiten Kupplungsteils mindestens zwei Nuten senkrecht zueinander eingeschnitten sind, deren Breite und Tiefe der Form des länglichen Zapfens der Antriebswelle entsprechen. Zur einfacheren Positionierung des zweiten Kupplungsteils auf dem Zapfen der Antriebswelle ist weiterhin vorgesehen, dass die durch die eingeschnittenen Nuten entstehenden Nocken eine axiale Kante zu den Nuten hin aufweisen. Beim Einsetzen des Kupplungsteils in das Handstück kann der Zapfen in eine der zwei Nuten des zweiten Kupplungsteils rutschen. Hierdurch ist eine schnelle und einfache Verbindung von Arbeitsteil und Antriebswelle gegeben.

Der Durchmesser des zweiten Kupplungsteils ist so bemessen, dass ein erster Durchmesser dem Innendurchmesser des ersten Kupplungsteils des verwendeten chirurgischen Werkzeuges entspricht, in dieses das zweite Kupplungsteil einsteckbar ist und, dass das zweite Kupplungsteil distal eine axiale Abschrägung aufweist, zum leichteren Einführen des zweiten Kupplungsteils in den Schaft des chirurgischen Werkzeuges. Weiterhin ist vorgesehen, dass ein zweiter Durchmesser des zweiten Kupplungsteiles größer ist als der erste Durchmesser, mit einer Stufe zum ersten Durchmesser, die als Stopper für das aufsteckbare Werkzeug dient und, dass ein dritter Durchmesser des zweiten Kupplungsteiles kleiner ist als der erste Durchmesser, wobei in die entstandene zylindrische Ausnehmung eine Dichtung aufnehmbar ist. Diese Ausgestaltungen gewährleisten, dass das zweite Kupplungsteil in einfachere und zuverlässigere Weise in den Schaft der chirurgischen Werkzeuge eingepasst werden kann, sowie zuverlässig auf der Antriebswelle des Antriebs sitzt.

In äußerst bevorzugter Ausgestaltung ist vorgesehen, dass zumindest ein Kupplungsteil aus Metall, vorzugsweise Edelstahl, und zumindest ein weiteres Kupplungsteil aus einem Mehrphasen-Kunststoff bestehen, wobei dieser aus einem Copolymer auf der Basis von Methacrylat und Styrol, vorzugsweise mit Butadiengehalt, besteht. Ein Kupplungsteil aus diesem Werkstoff ist einfach herzustellen, kann in Form gespritzt werden und weist den weiteren Vorteil auf, dass dieser Werkstoff sich während eines Sterilisationsprozesses derart verformt, dass für das verwendete chirurgische Werkzeug keine Passung mehr in einem Handstück gegeben ist, wodurch das Problem der Reinigung und Sterilisation dieser Werkzeuge gänzlich entfällt. Bei jeder Knochenbearbeitung ist damit ein optimal scharfes Werkzeug sichergestellt.

Weitere Vorteile und Merkmale der Erfindung ergeben sich aus den Ansprüchen und aus der nachfolgenden Beschreibung, in der ein Ausführungsbeispiel der Erfindung unter Bezugnahme auf die Zeichnungen im Einzelnen erläutert ist. Dabei zeigt:
- Fig. 1: einen Längsschnitt durch ein erfindungsgemäßes chirurgisches Instrument, wobei ein Werkzeug in einem Handstück verrastet ist;
- Fig. 2: einen teilweisen Längsschnitt eines erfindungsgemäßen chirurgischen Instrumentes nach Schnitt A-A gemäß Fig. 3;
- Fig. 3: eine Stirnansicht auf die Einführungsöffnung des Handstückes des erfindungsgemäßen chirurgischen Instruments;
- Fig. 4a: eine Seitenansicht auf ein zweites Kupplungsstück des erfindungsgemäßen chirurgischen Instruments;
- Fig. 4b: eine stirnseitige Ansicht eines proximalen Endes des zweiten Kupplungsteiles;
- Fig. 4c: ein Längsschnitt durch das zweite Kupplungsteil nach Schnitt B-B gemäß Fig. 4a;
- Fig. 5: einen Satz an chirurgischen Werkzeugen zur Verwendung mit dem erfindungsgemäßen chirurgischen Instrument.

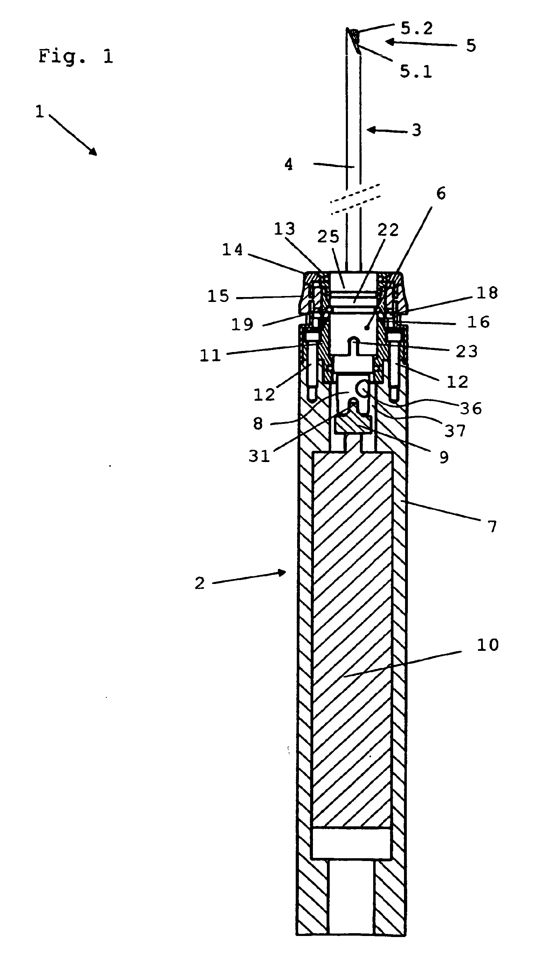

Die Fig. 1 zeigt ein erfindungsgemäßes chirurgisches Instrument 1 in einem Längsschnitt. Das chirurgische Instrument 1 weist ein im Wesentlichen zylindrisches Handstück 2 aus Edelstahl auf, in welches ein chirurgisches Werkzeug 3, insbesondere ein Knochenfräser, zerstörungsfrei lösbar einsetzbar ist. Das Werkzeug 3 weist ein äußeres zylindrisches Rohr 4 und ein in diesem drehbares Arbeitsteil 5 auf. Das Arbeitsteil 5 ist aus einem zylindrischen Schaft 5.1 und einem Werkzeugkopf 5.2 gebildet. Das Rohr 4 ist mittels des ersten Kupplungsteiles 6 mit einer fest am außen zylindrischen Griff 7, des Handstücks 2 angebrachten Aufnahme 11 verbindbar. Das Arbeitsteil 5 ist über ein zweites Kupplungsteil 8 mit einer Arbeitswelle in Form eines Zapfens 31 eines im Handstück 2 angeordneten Antriebs 9 verbindbar und gleichzeitig innerhalb des Rohres 4 drehbar gelagert. Der Antrieb 9 ist durch einen Motor 10, vorzugsweise ein Elektromotor, antreibbar.

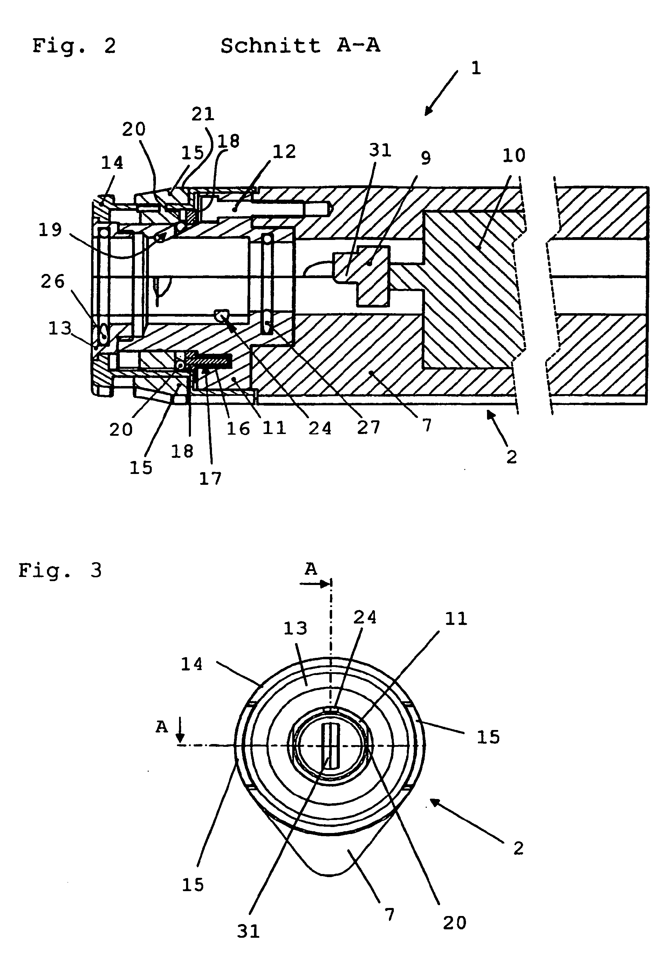

Der zylindrisch geformte Griff 7 des Handstücks 2 ist mit einer ebenfalls zylindrisch ausgebildeten Aufnahme 11 mittels zweier Zylinderschrauben 12 verbunden. Das Handstück 2 weist distal stirnseitig einen mit einem Gewinde versehenen Verschlussring 13 und eine diesen umgebende Endkappe 14 auf. Entlang des äußeren Randes der Aufnahme 11 ist ein Betätigungsschieber 15 axial entgegen der Wirkung von Federn 16 verschiebbar, welche jeweils in einer Ausnehmung 17 innerhalb der Aufnahme 11 angeordnet sind (Fig. 2). In der vorliegenden Ausgestaltung sind vier Federn 16 einander gegenüberliegend im Gehäuse der Aufnahme 11 vorgesehen. Ihr Widerlager finden die Federn 16 im dargestellten Ausführungsbeispiel der Fig. 1 und 2 einerseits proximal in der Ausnehmung 17 des Handstücks 2 und andererseits distal an einer ringförmigen bewegbaren Anpressplatte 18, sodass die Federn 16 diese Anpressplatte 18 in distale Richtung vorbelasten bzw. drücken.

Fig. 2 zeigt einen weiteren teilweisen Längsschnitt des chirurgischen Instruments 1, gemäß Schnitt A-A nach Fig. 3. In zwei gegenüberliegenden länglich, radial schräg in die Innenwand der Aufnahme 11 eingefrästen Nuten 19 befinden sich zwei Klemmstifte 20, wobei die Klemmstifte 20 in einer Verriegelungsposition kreissehnenförmig in das Innere der Aufnahme 11 ragen. Hierbei ist sichtbar, dass die federbelastete Anpressplatte 18 durch die Federn 16 gegen die Klemmstifte 20 gedrückt ist. Der Betätigungsschieber 15 ist hierbei in einer Freigabeposition dargestellt, wobei der Betätigungsschieber 15 einen Anschlag an einer Schulter 21 der Endkappe 14 findet.

Fig. 1 zeigt, dass der innere Durchmesser der Aufnahme 11 derart gestaltet ist, das auswechselbare chirurgische Werkzeuge 3, deren rundes Rohr 4 ein kraft- und/oder stoffschlüssig aufgebrachtes erstes Kupplungsteil 6 aus Metall aufweist, mit geringem Spiel in die Aufnahme 11 einführbar sind. Ein metallenes Kupplungsteil 6 dient zur sicheren Führung und Befestigung innerhalb der Aufnahme. Das erste Kupplungsteil 6 ist hierbei zylindrisch ausgebildet und weist eine umlaufende Ringnut 22 auf, in welche die Klemmstifte 20 in einer Verriegelungsposition eingreifen. Um ein Mitdrehen des Rohres 4 und einen ordnungsgemäßen Sitz des Werkzeugs 3 innerhalb des Handstücks 2 zu gewährleisten, ist weiterhin proximal am ersten Kupplungsteil 6 ein Führungsschlitz 23 vorgesehen, welcher über einen Verdrehschutz 24 innerhalb der Aufnahme 11 eingreift. Das erste Kupplungsteil 6 weist eine weitere umlaufende Nut 25 auf, die mit einem farbigen Ring bestückbar ist, um die chirurgischen Werkzeuge 3 besser zu unterscheiden. Um eine Abdichtung des verwendeten chirurgischen Werkzeuges 3 innerhalb des Handstückes 2 zu gewährleisten, sind Ringdichtungen an zwei Positionen vorgesehen. Zum Einen ist eine Ringdichtung 26 zur Abdichtung der Aufnahme 11 des Werkzeuges 3 nach außen hin im Verschlussring 13 im Handstück 2 vorgesehen. Zum Anderen ist eine weitere Ringdichtung 27 zur Abdichtung des ersten Kupplungsteiles 6 zum Antrieb 9 hin innerhalb der Aufnahme 11 angeordnet.

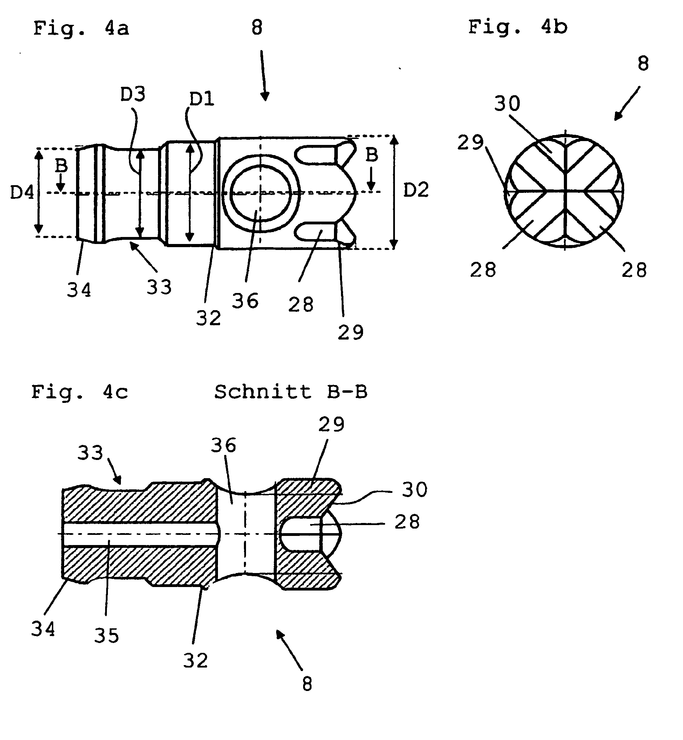

Die Figuren 4a bis 4c zeigen eine bevorzugte Ausgestaltung des in das Rohr 4 einsteckbaren zweiten Kupplungsteiles 8. Das zweite Kupplungsteil 8 zeigt eine zylindrisch ausgebildete Form, und besteht aus Kunststoff, wobei der Kunststoff zumindest einen Teil Methacrylat-Styrol und einen Anteil Butadien enthält. Die Fig. 4a zeigt hierbei eine seitliche Ansicht des zweiten Kupplungsteiles 8, die, wie auch die stirnseitige Ansicht 4b beschreibt, zwei kreuzweise eingeschnittene Nuten 28 zeigt. Die Nuten 28 stehen, wie in der Stirnansicht der Fig. 4b dargestellt, senkrecht aufeinander. Die dadurch entstehenden Nocken 29 weisen weiterhin abgeschrägte Kanten 30 auf, die ein leichtes Aufsetzen des zweiten Kupplungsteils 8 auf die Antriebswelle in Form eines Zapfens 31 gewährleistet. Das zweite Kupplungsteil 8 weist einen ersten Durchmesser D1 auf, der so bemessen ist, dass er dem inneren Durchmesser eines Kupplungsteils 6 eines verwendeten Werkzeuges 3 entspricht und somit das zweite Kupplungsteil 8 in dieses Kupplungsteil 6 einsteckbar ist. Des Weiteren ist ein zweiter Durchmesser D2 vorgesehen, der größer ist als der erste Durchmesser D1, wobei eine Stufe 32 zum ersten Durchmesser D1 als Anschlag für das aufsteckbare chirurgische Werkzeug 3 dient. Ein dritter Durchmesser D3 des zweiten Kupplungsteils 8 ist weiterhin kleiner als der erste Durchmesser D1 ausgebildet, wobei in die entstehende zylinderförmige Ausnehmung 33 zum Beispiel eine Dichtung aufnehmbar ist. Das zweite Kupplungsteil 8 weist distal eine axiale Abschrägung 34 auf, die von einem ersten Durchmesser D1 auf einen vierten Durchmesser D4 abfallend ausgebildet ist, zum besseren Einführen des zweiten Kupplungsteils 8 in das Kupplungsteils 6 des chirurgischen Werkzeuges 3. Im Inneren des zweiten Kupplungsteiles 8 sind weiterhin zwei senkrecht zueinander stehenden Bohrungen vorgesehen, wobei eine axiale Bohrung 35 durch eine radiale durchgängige Bohrung 36 durchbrochen ist, wie in Fig. 4c dargestellt ist. Weiter kann über beide ein Absaugen von ausgefrästem Knochmaterial erfolgen. Hierzu führt durch die Wandung des Griffs 7 und den Hohlraum 37 eine Querbohrung, an die ein Absaugschlauch angeschlossen werden kann, über den dann die Absaugung erfolgt. Die axiale Bohrung 35 dient zur Aufnahme des Schaftes 5.1 des chirurgischen Werkzeuges 3.

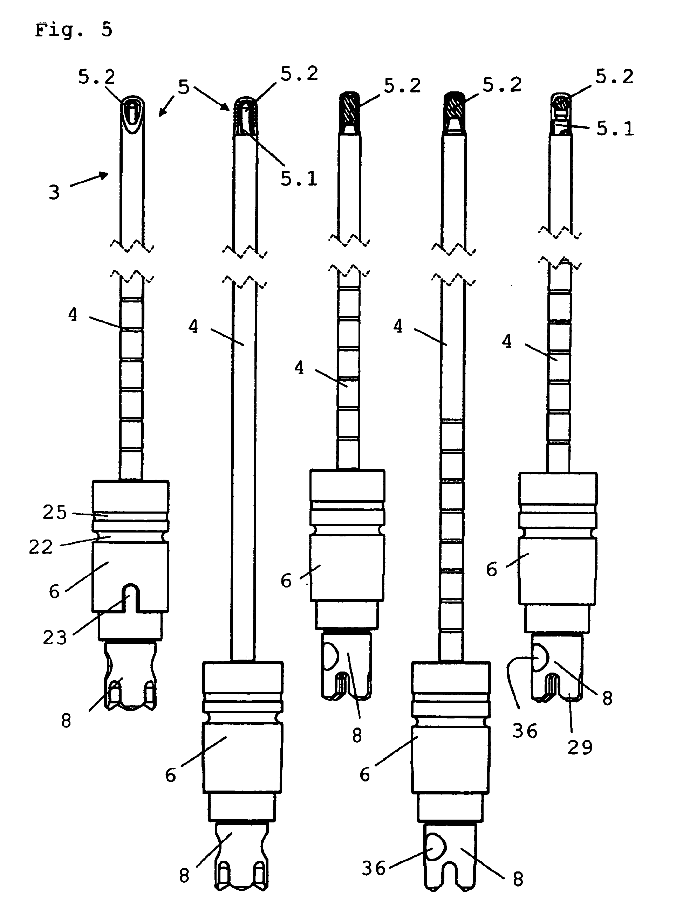

Die Fig. 5 zeigt beispielhaft einen Satz von chirurgischen Werkzeugen 3, insbesondere Fräser oder Bohrer für die Wirbelsäulenchirurgie, mit jeweils einem Rohr 4 mit einem distal aufgesetzten ersten Kupplungsteils 6 und einem innerhalb des Rohres 4 angeordneten drehbaren Arbeitsteils 5 mit einem Schaft 5.1, dessen distales Ende innerhalb eines zweiten Kupplungsteils 8 fest verbunden (gegebenenfalls umspritzt) ist. Das zweite Kupplungsteil 8 ist hierbei drehbar innerhalb des Rohres 4 gelagert. Durch die Kombination dieser Kupplungsteile kann für das erfindungsgemäße chirurgische Instrument 1 eine Vielzahl an unterschiedlichen chirurgischen Werkzeugen 3 verwendet werden.

Ein entsprechendes chirurgisches Werkzeug 3 wird nun wie folgt mit dem Handstück 2 verwendet:

Das chirurgische Werkzeug 3 wird vorsichtig in die Aufnahme 11 des Handstücks 2 geführt. Hierbei werden die Klemmstifte 20 innerhalb ihrer Nuten 19 in Richtung der Anpressplatte 18 bewegt und die Anpressplatte 18 entgegen der Federwirkung der Federn 16 proximal verschoben.
Das erste Kupplungsteil 6 wird weiter in die Aufnahme 11 des Handstückes 2 eingeführt, und hierbei leicht gedreht, sodass der Führungsschlitz 23 des ersten Kupplungsteiles 6 über den Verdrehschutz 24 geschoben wird. Gleichzeitig bewirkt eine leichte Drehung des Werkzeuges 3, dass das zweite Kupplungsteil 8 mittels einer der Nuten 28 über den Zapfen 31 geschoben wird, und somit in die Antriebswelle 9 greift. Sitzt das zweite Kupplungsteil 6 ordnungsgemäß auf dem Zapfen 31, liegen die Ringnut 22 und die Klemmstifte 20 auf gleicher Höhe, so dass diese in die Ringnut 22 des ersten Kupplungsteiles 6 eingreifen können. Die Anpressplatte 18 wird durch die Federbelastung der Federn 16 distal nach vorne verschoben. Hierdurch wird der Betätigungsschieber 15 unter Federwirkung in eine Verriegelungsposition axial in Richtung des distalen Endes des Handstückes 2 hin verschoben. Zum Lösen der Verbindung wird der Betätigungsschieber 15 in eine Öffnungsposition axial in proximale Richtung des Handstückes 2 verschoben, wobei der Betätigungsschieber 15 die Anpressplatte 18 und somit die Federn 16 ebenfalls in proximale Richtung verschiebt bzw. zusammendrückt. Hierbei werden die Klemmstifte 20 innerhalb ihrer Nuten 19 in Richtung der Anpressplatte 18 bewegt und geben den Weg frei für das Entfernen des chirurgischen Werkzeuges 3. Durch eine doppelte Kupplung eines chirurgischen Werkzeuges 3, wobei zum Einen eine Klemmstiftbefestigung ein erstes Kupplungsteil eines Rohres 4 eines chirurgischen Werkzeuges 3 ergreift und zum Anderen ein zweites Kupplungsteil 8 eine drehbare Verbindung zwischen einem Schaft 5.1 eines Arbeitsteils 5 und einer Antriebswelle in Form eines Zapfens 31 eines Antriebs 9 gewährleistet, ist eine sichere Kupplung zwischen einem chirurgischen Werkzeug und einem entsprechenden Handstück 2 gegeben.

### Bezugszeichenliste

- 1: Chirurgisches Instrument
- 2: Handstück
- 3: chirurgisches Werkzeug
- 4: Rohr
- 5: Arbeitsteil
- 5.1: Schaft
- 5.2: Werkzeugkopf
- 6: zweites Kupplungsteil
- 7: Griff
- 8: erstes Kupplungsteil
- 9: Antrieb
- 10: Motor
- 11: Aufnahme
- 12: Zylinderschraube
- 13: Verschlussring
- 14: Endkappe
- 15: Betätigungsschieber
- 16: Feder
- 17: Ausnehmung
- 18: Anpressplatte
- 19: Nut
- 20: Klemmstifte
- 21: Schulter
- 22: Ringnut
- 23: Führungsschlitz
- 24: Verdrehschutz
- 25: Ringnut
- 26: Ringdichtung
- 27: Ringdichtung
- 28: Nut
- 29: Nocken
- 30: Kante
- 31: Zapfen
- 32: Stufe
- 33: Ausnehmung
- 34: Abschrägung
- 35: axiale Bohrung
- 36: radiale Bohrung
- 37: Hohlraum
- D1: erster Durchmesser
- D2: zweiter Durchmesser
- D3: dritter Durchmesser
- D4: vierter Durchmesser

## Patentansprüche

1. Chirurgisches Instrument mit einem einen Antrieb (9) aufweisenden Handstück (2) und mit einem chirurgischen Werkzeug (3), welches ein Arbeitsteil (5) mit einem Schaft (5.1) und einem Werkzeugkopf (5.2) aufweist, wobei ein Rohr (4) des chirurgischen Werkzeugs (3) mit dem Handstück (2) mittels eines ersten Kupplungsteiles (6) lösbar verbindbar ist, wobei eine Aufnahme (11) im Handstück (2) zum Aufnehmen des ersten Kupplungsteils (6) des Werkzeugs (3) vorgesehen ist, wobei der Schaft (5.1) des Arbeitsteils (5) mit einer Antriebswelle in Form eines Zapfens (31) des Antriebs (9) mittels eines zweiten Kupplungsteiles (8) lösbar verbindbar ist, wobei das Werkzeug ein Rohr (4) aufweist, wobei das Arbeitsteil (5) sich im Rohr (4) befindet, wobei das Arbeitsteil (5) drehbar gelagert ist, wobei das Rohr (4) mit Handstück (2) und Arbeitsteil (5) mit Antriebswelle miteinander gleichzeitig verbindbar sind, wobei das Handstück (2) zwei in länglichen radialschräg in der Innenwand der Aufnahme angeordneten Nuten (19) verschiebbare Klemmstifte (20) aufweist, wobei die Klemmstifte (20) quer unter Federwirkung kreissehnenförmig in das Innere der Aufnahme (11) ragen, und wobei beim Einführen des Werkzeugs (3) die Klemmstifte (20) entgegen der Federwirkung in den schrägen Nuten (19) verschiebbar sind.

2. Chirurgisches Instrument nach Anspruch 1, **dadurch gekennzeichnet, dass** das chirurgische Werkzeug (3) ein hohles zylindrisches Rohr (4) und ein darin befindliches Arbeitsteil (5) mit einem Schaft (5.1) und einem Werkzeugkopf (5.2) aufweist, wobei das Arbeitsteil (5) drehbar gelagert ist.

3. Chirurgisches Instrument nach Anspruch 1 oder 2, **dadurch gekennzeichnet, dass** das erste Kupplungsteil (6) proximal am Rohr (4) des chirurgischen Werkzeuges (3) angeordnet ist.

4. Chirurgisches Instrument nach einem der Ansprüche 1 bis 3, **dadurch gekennzeichnet, dass** das Handstück (2) einen Griff (7), mit einer Aufnahme (11) für das Werkzeug (3) und eine Arretierung innerhalb der Aufnahme (11) aufweist und/oder dass am Handstück (2) distal ein Verschlussring (13) eingeschraubt und mit einer aufgesteckten Endkappe (14) verschlossen ist.

5. Chirurgisches Instrument nach einem der vorangehenden Ansprüche, **dadurch gekennzeichnet, dass** am Handstück (2) ein Betätigungsschieber (15) angeordnet ist, der mittels einer ringförmigen Anpressplatte (18) mit wenigstens einer Feder (16) beaufschlagt ist und/oder insbesondere in eine Verriegelungsposition und eine Freigabeposition axial verschiebbar ist und/oder zumindest mit einer Anpressplatte (18) ringförmig ausgebildet ist.

6. Chirurgisches Instrument nach einem der vorhergehenden Ansprüche, **dadurch gekennzeichnet, dass** ein Durchmesser der Aufnahme (11) dem Durchmesser der auswechselbaren Werkzeuge (3) angepasst ist, wobei das proximal am Werkzeug (3) angeordnete erste Kupplungsteil (6) mit einer einer Arretierung zugeordneten ringförmigen Nute (19) versehen ist.

7. Chirurgisches Instrument nach einem der vorherigen Ansprüche, **dadurch gekennzeichnet, dass** das erste Kupplungsteil (6) einen Führungsschlitz (23) aufweist, der der Form eines Verdrehschutzes (24) innerhalb der Aufnahme (11) entsprechend ausgebildet ist.

8. Chirurgisches Instrument nach einem der vorhergehenden Ansprüche, **dadurch gekennzeichnet, dass** im Verschlussring (13) des Handstücks (2) eine Ringdichtung (26) zur Abdichtung der Aufnahme (11) des Werkzeugs vorgesehen ist und/oder dass in der Aufnahme (11) eine Ringdichtung (27) zur Abdichtung des ersten Kupplungsteils (6) zur Antriebswelle (9) vorgesehen ist.

9. Chirurgisches Instrument nach einem der vorhergehenden Ansprüche, **dadurch gekennzeichnet, dass** das Handstück (2) eine Antriebswelle (9) eines Antriebs (10) mit einem Zapfen (31) aufweist, der in das zweite Kupplungsteil (8) eingreift.

10. Chirurgisches Instrument nach einem der vorhergehenden Ansprüche, **dadurch gekennzeichnet, dass** das zweite Kupplungsteil (8) im Wesentlichen zylindrisch mit einer axialen Bohrung (35) ausgebildet ist, wobei der Schaft (5.1) des Arbeitsteils (5) proximal innerhalb der axialen Bohrung (35) fest verbunden ist und dass insbesondere das zweite Kupplungsteil (8) radial eine durchgängige radiale Bohrung (36) hat, die senkrecht zur axialen Bohrung (35) angeordnet ist und diese abschließt.

11. Chirurgisches Instrument nach einem der vorhergehenden Ansprüche, **dadurch gekennzeichnet, dass** im Endbereich des zweiten Kupplungsteils (8) proximal mindestens zwei Nuten (28) senkrecht zueinander eingeschnitten sind, deren Breite und Tiefe der Form des länglichen Zapfens (31) des Antriebs (9) entsprechen.

12. Chirurgisches Instrument nach Anspruch 11, **dadurch gekennzeichnet, dass** durch die eingeschnittenen Nuten (28) entstehende Nocken (29) zur einfacheren Positionierung des zweiten Kupplungsteils (8) auf den Zapfen (31) des Antriebs (9) jeweils eine axiale Kante zu den Nuten (28) hin aufweisen.

13. Chirurgisches Instrument nach einem der vorhergehenden Ansprüche, **dadurch gekennzeichnet, dass** das zweite Kupplungsteil (8) im Durchmesser so bemessen ist, dass ein erster Durchmesser (D1) einem Innendurchmesser des ersten Kupplungsteils (6) des verwendeten Werkzeuges (3) entspricht und in diesen das zweite Kupplungsteil (8) einsteckbar ist und/oder ein zweiter Durchmesser (D2) des zweiten Kupplungsteiles (8) größer ist als der erste Durchmesser (D1), mit einer Stufe zum ersten Durchmesser (D1), die als Anschlag für das aufsteckbare Werkzeug (3) dient und/oder ein dritter Durchmesser (D3) des zweiten Kupplungsteiles (8) kleiner ist als der erste Durchmesser (D1), wobei in die entstandene Zylinderausnehmung eine Dichtung aufnehmbar ist.

14. Chirurgisches Instrument nach einem der vorhergehenden Ansprüche, **dadurch gekennzeichnet, dass** das erste Kupplungsteil (6) und/oder das zweite Kupplungsteil (8) zumindest einen Bereich aufweisen, der aus einem Mehrphasen-Kunststoff besteht, wobei insbesondere der Bereich ein Copolymer auf der Basis von Methacrylat und Styrol besteht, sowie teils Butadien enthält.

15. Satz von Chirurgischen Werkzeugen mit einem chirurgischen Instrument zur lösbaren Verbindung eines Handstückes (2) mit einem chirurgischen Werkzeug (3), nach einem der Ansprüche 1 bis 14.

## Claims

1. Surgical instrument comprising a handpiece (2) with a drive (9), and comprising a surgical tool (3) that has a working part (5) with a shank (5.1) and with a tool head (5.2), wherein the tube (4) of the surgical tool (3) can be detachably connected to the handpiece (2) by means of a first coupling part (6), wherein a seat (11) in the handpiece (2) is provided for receiving the first coupling part (6) of the tool (3), wherein the shank (5.1) of the working part (5) can be detachably connected to a drive shaft in the form of a pin (31) of the drive (9) by means of a second coupling part (8), wherein the tool has a tube (4), wherein the working part (5) is located in the tube (4), wherein the working part (5) is rotatably mounted, wherein the tube (4) of the surgical tool (3) can be detachably connected to the handpiece (2) by means of a second coupling part (6), wherein a seat (11) in the handpiece (2) is provided for receiving the second coupling part (6) of the tool (3), wherein the tube (4) can be connected to handpiece (2) and the working part (5) to drive shaft simultaneously with each other, wherein the handpiece (2) has two clamping pins (20) that are movable in elongate grooves (19) arranged radially obliquely in the inner wall of the seat, wherein the clamping pins (20), under a spring action, protrude transversely and in a chord shape into the interior of the seat (11), and wherein the clamping pins (20), upon insertion of the tool (3), are movable in the oblique grooves (19) counter to the spring action.

2. Surgical instrument according to Claim 1, **characterized in that** the surgical tool (3) has a hollow cylindrical tube (4) and, located in the latter, a working part (5) with a shank (5.1) and with a tool head (5.2), wherein the working part (5) is rotatably mounted.

3. Surgical instrument according to Claim 1 or 2, **characterized in that** the first coupling part (6) is arranged proximally on the tube (4) of the surgical tool (3).

4. Surgical instrument according to one of Claims 1 to 3, **characterized in that** the handpiece (2) has a grip (7), with a seat (11) for the tool (3), and a locking mechanism inside the seat (11), and/or **in that** a closure ring (13) is screwed in distally on the handpiece (2) and is closed by a plugged-on end cap (14).

5. Surgical instrument according to one of the preceding claims, **characterized in that** an actuating slide (15), arranged on the handpiece (2), is subjected to the action of at least one spring (16) by means of an annular pressure plate (18) and/or is axially movable in particular to a locking position and a release position and/or is designed in a ring shape at least with a pressure plate (18).

6. Surgical instrument according to one of the preceding claims, **characterized in that** a diameter of the seat (11) is adapted to the diameter of the exchangeable tools (3), wherein the first coupling part (6) arranged proximally on the tool (3) is provided with an annular groove (19) assigned to a locking mechanism.

7. Surgical instrument according to one of the preceding claims, **characterized in that** the first coupling part (6) has a guide slit (23), which is designed corresponding to the shape of an anti-rotation means (24) inside the seat (11).

8. Surgical instrument according to one of the preceding claims, **characterized in that**, in the closure ring (13) of the handpiece (2), an annular seal (26) is provided for sealing the seat (11) of the tool, and/or **in that** an annular seal (27) for sealing the first coupling part (6) relative to the drive shaft (9) is provided in the seat (11).

9. Surgical instrument according to one of the preceding claims, **characterized in that** the handpiece (2) has a drive shaft (9) of a drive (10) with a pin (31), which engages in the second coupling part (8).

10. Surgical instrument according to one of the preceding claims, **characterized in that** the second coupling part (8) has a substantially cylindrical design with an axial bore (35), wherein the shank (5.1) of the working part (5) is firmly connected proximally inside the axial bore (35), and **in that** in particular the second coupling part (8) has in the radial direction a continuous radial bore (36), which is arranged perpendicular to the axial bore (35) and closes the latter off.

11. Surgical instrument according to one of the preceding claims, **characterized in that**, in the proximal end area of the second coupling part (8), at least two grooves (28) are cut in perpendicular to each other, their width and depth corresponding to the shape of the elongate pin (31) of the drive (10).

12. Surgical instrument according to Claim 11, **characterized in that** cams (29) resulting from the cutin grooves (28) each have an axial edge facing towards the grooves (28), in order to permit easier positioning of the second coupling part (8) onto the pin (31) of the drive (9).

13. Surgical instrument according to one of the preceding claims, **characterized in that** the second coupling part (8) is dimensioned in diameter in such a way that a first diameter (D1) corresponds to an internal diameter of a first coupling part (6) of the used tool (3) and the second coupling part (8) can be plugged into this, and/or a second diameter (D2) of the second coupling part (8) is greater than the first diameter (D1), with a step to the first diameter (D1) serving as abutment for the plug-on tool (3), and/or a third diameter (D3) of the second coupling part (8) is smaller than the first diameter (D1), it being possible for a seal to be received in the resulting cylinder recess.

14. Surgical instrument according to one of the preceding claims, **characterized in that** the first coupling part (6) and/or the second coupling part (8) have at least one area made of a multi-phase plastic, wherein in particular the area is composed of a copolymer based on methacrylate and styrene and in part contains butadiene.

15. Set of surgical tools with a surgical instrument for detachably connecting a handpiece (2) to a surgical tool (3), according to one of Claims 1 to 14.

## Revendications

1. Instrument chirurgical doté d'une pièce à main (2) comportant un entraînement (9) et d'un outil chirurgical (3) comportant une partie fonctionnelle (5) dotée d'un manche (5.1) et d'une tête d'outil (5.2), le tube (4) de l'outil chirurgical (3) pouvant être relié de façon amovible à la pièce à main (2) par le biais d'une première partie de couplage (6), le manche (5.1) de la partie fonctionnelle (5) pouvant être raccordé de façon amovible à un arbre d'entraînement prenant la forme d'un tenon (31) de l'entraînement (10) par le biais d'une deuxième partie de couplage (8), l'outil comportant un tube (4), la partie fonctionnelle (5) se trouvant dans le tube (4), la partie fonctionnelle (5) étant disposée de façon à pouvoir pivoter, un logement (11) étant prévu dans la pièce à main (2) pour recevoir la première partie de couplage (6) de l'outil (3), le tube (4) doté de la pièce à main (2) et la partie fonctionnelle (5) dotée de l'arbre d'entraînement (9) pouvant être raccordés simultanément l'un à l'autre, la pièce à main (2) comportant deux tiges de serrage (20) pouvant être coulissées dans des rainures (19) oblongues disposées à l'oblique dans le plan radial dans la paroi intérieure du logement, les tiges de serrage (20) saillant transversalement en forme de cordon circulaire à l'intérieur du logement (11) sous l'effet d'un ressort et les tiges de serrage (20) pouvant être déplacées dans les rainures (19) obliques contre l'effet du ressort lors de l'introduction de l'outil (3).

2. Instrument chirurgical selon la revendication 1, **caractérisé en ce que** l'outil chirurgical (3) comporte un tube (4) cylindrique creux et une partie fonctionnelle (5) placée à l'intérieur dotée d'un manche (5.1) et d'une tête d'outil (5.2), la partie fonctionnelle (5) étant disposée de façon à pouvoir pivoter.

3. Instrument chirurgical selon la revendication 1 ou 2, **caractérisé en ce que** la première partie de couplage (6) est disposée de façon proximale contre le tube (4) de l'outil chirurgical (3).

4. Instrument chirurgical selon l'une quelconque des revendications 1 à 3, **caractérisé en ce que** la pièce à main (2) comporte une poignée (7), avec un logement (11) pour l'outil (3) et une butée à l'intérieur du logement (11), et/ou qu'une bague de fermeture (13) est vissée dans le plan distal contre la pièce à main (2) et fermée avec un bouchon terminal (14) enfiché dessus.

5. Instrument chirurgical selon l'une quelconque des revendications précédentes, **caractérisé en ce qu'**un piston d'actionnement (15) est disposé contre la pièce à main (2), ledit piston étant sollicité à l'aide d'une plaque de compression (18) de forme annulaire dotée d'au moins un ressort (16) et/ou pouvant notamment être déplacé dans le plan axial dans une position de verrouillage et une position de libération et/ou prenant au moins une forme annulaire avec une plaque de compression (18).

6. Instrument chirurgical selon l'une quelconque des revendications précédentes, **caractérisé en ce qu'**un diamètre du logement (11) est adapté au diamètre des outils (3) interchangeables, la première partie de couplage (6) disposée dans le plan proximal contre l'outil (3) étant pourvue d'une rainure (19) de forme annulaire associée à une butée.

7. Instrument chirurgical selon l'une quelconque des revendications précédentes, **caractérisé en ce que** la première partie de couplage (6) comporte une fente de guidage (23) réalisée de façon correspondante à la forme d'une protection antitorsion (24) placée à l'intérieur du logement (11).

8. Instrument chirurgical selon l'une quelconque des revendications précédentes, **caractérisé en ce qu'**un joint annulaire (26) est prévu dans la bague de fermeture (13) de la pièce à main (2) pour étanchéifier le logement (11) de l'outil et/ou qu'un joint annulaire (27) est prévu dans le logement (11) pour étanchéifier la première partie de couplage (6) par rapport à l'arbre d'entraînement (9).

9. Instrument chirurgical selon l'une quelconque des revendications précédentes, **caractérisé en ce que** la pièce à main (2) comporte un arbre d'entraînement (9) d'un entraînement (10) doté d'un tenon (31) s'engrenant dans la deuxième partie de couplage (8).

10. Instrument chirurgical selon l'une quelconque des revendications précédentes, **caractérisé en ce que** la deuxième partie de couplage (8) est réalisée pour l'essentiel de façon cylindrique avec un alésage (35) axial, le manche (5.1) de la partie fonctionnelle (5) étant raccordé de façon permanente dans le plan proximal à l'intérieur de l'alésage (35) axial et que la deuxième partie de couplage (8) a notamment dans le plan radial un alésage (36) radial traversant disposé perpendiculairement à l'alésage (35) axial et le terminant.

11. Instrument chirurgical selon l'une quelconque des revendications précédentes, **caractérisé en ce qu'**au moins deux rainures (28) se croisent perpendiculairement l'une par rapport à l'autre dans le plan proximal dans la zone d'extrémité de la deuxième partie de couplage (8), la largeur et la profondeur desdites rainures épousant la forme du tenon (31) oblong de l'entraînement (9).

12. Instrument chirurgical selon la revendication 11, **caractérisé en ce que** des cames (29) sortant des rainures (28) se croisant comportent respectivement une arête axiale orientée vers les rainures (28) en vue de simplifier le positionnement de la deuxième partie de couplage (8) sur le tenon (31) de l'entraînement (9).

13. Instrument chirurgical selon l'une quelconque des revendications précédentes, **caractérisé en ce que** la deuxième partie de couplage (8) présente un diamètre tel qu'un premier diamètre (D1) correspond à un diamètre intérieur de la première partie (6) de l'outil (3) utilisé et que la deuxième partie de couplage (8) peut être enfiché dans celui-ci et/ou un deuxième diamètre (D2) de la deuxième partie de couplage (8) supérieur au premier diamètre (D1), avec un cran par rapport au premier diamètre (D1) servant de butée pour l'outil (3) enfiché dessus et/ou un troisième diamètre (D3) de la deuxième partie de couplage (8) inférieur au premier diamètre (D1), un joint pouvant être reçu dans l'évidement cylindrique crée.

14. Instrument chirurgical selon l'une quelconque des revendications précédentes, **caractérisé en ce que** la première partie de couplage (6) et/ou la deuxième partie de couplage (8) comportent au moins une région composée de matière plastique polyphasée, la région contenant notamment un copolymère à base de méthacrylate et de styrène, ainsi qu'une partie de butadiène.

15. Jeu d'outils chirurgicaux doté d'un instrument chirurgical pour le raccordement amovible d'une pièce à main (2) avec un outil chirurgical (3), selon l'une quelconque des revendications 1 à 14.
